(19)

Europäisches Patentamt
European Patent Office
Office européen des brevets

(11)  EP 4 575 569 A2

(12)  **EUROPEAN PATENT APPLICATION**

(43) Date of publication:
25.06.2025 Bulletin 2025/26

(21) Application number: 24220634.0

(22) Date of filing: 17.12.2024

(51) International Patent Classification (IPC):
*G01S 7/52* (2006.01)   *G01S 15/89* (2006.01)
*A61B 8/00* (2006.01)

(52) Cooperative Patent Classification (CPC):
**G01S 15/8988; G01S 7/52085; G01S 15/8927;
G01S 15/8981;** A61B 8/488; A61B 8/54;
G01S 15/8963

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 20.12.2023 JP 2023214738

(71) Applicant: FUJIFILM Corporation
Tokyo 106-8620 (JP)

(72) Inventors:
• **TANAKA, Tomohiko**
Tokyo, 106-8620 (JP)

• **SHIMIZU, Motochika**
Tokyo, 106-8620 (JP)
• **YASUDA, Jun**
Tokyo, 106-8620 (JP)
• **YOSHIKAWA, Hideki**
Tokyo, 106-8620 (JP)
• **KURIBARA, Hiroshi**
Tokyo, 106-8620 (JP)

(74) Representative: **Dehns Germany Partnerschaft
mbB**
**Theresienstraße 6-8**
**80333 München (DE)**

(54)  **ULTRASOUND DIAGNOSTIC APPARATUS**

(57)   An object of the present disclosure is to reduce the number of times ultrasound pulses are transmitted in Doppler measurement while suppressing unnecessary components included in information obtained by the Doppler measurement.

An information processing unit (26) includes a Doppler measurement section (40) that generates Doppler measurement information based on a reception Doppler signal output from a wall filter (38), and a machine learning model (44) constructed based on training information and target information. The training information is information obtained from K reception Doppler signals. The target information is information obtained from J reception Doppler signals for the same measurement target object as that obtained from the K reception Doppler signals. Here, J and K are integers of 2 or more, and J is greater than K. The machine learning model (44) generates the Doppler measurement information based on input information. The input information is information generated from the K reception Doppler signals for a measurement target object in a subject.

FIG. 1

EP 4 575 569 A2

**Description**

**BACKGROUND OF THE INVENTION**

1. Field of the Invention

The present disclosure relates to an ultrasound diagnostic apparatus, and particularly, to an apparatus that performs Doppler measurement.

2. Description of the Related Art

An ultrasound diagnostic apparatus that observes a subject by transmitting and receiving ultrasound waves is widely used. An operation mode of the ultrasound diagnostic apparatus includes a Doppler mode in which a blood flow velocity at an observation site is measured. In the measurement in the Doppler mode, ultrasound pulses are transmitted to the subject a plurality of times, and reflected waves generated a plurality of times in the subject are received. A plurality of reception signals (hereinafter, referred to as reception Doppler signals) having Doppler frequency components are generated from a plurality of reception pulse signals based on the reflected waves received a plurality of times. Further, high-pass filter processing (wall filter processing) is performed on the plurality of reception Doppler signals, and the blood flow velocity in a region of interest set on an ultrasound beam formed by the ultrasound pulses is obtained based on the plurality of reception Doppler signals after the high-pass filter processing.

A filter such as a finite impulse response filter (FIR filter) or an infinite impulse response filter (IIR filter) is used as a high-pass filter. As the number of reception Doppler signals to be subjected to the high-pass filter processing increases, the number of tap coefficients of the high-pass filter can be increased. Therefore, the greater the number of reception Doppler signals to be subjected to the high-pass filter processing is, the steeper filter characteristics can be made. This allows for a greater attenuation amount in a low-frequency range while ensuring the high-pass frequency band. As a result, noise such as clutter in a displayed ultrasound image is suppressed.

As technologies related to the disclosure of the present application, the following JP2005-102718A, JP1996-154935A (JP-H08-154935A), and JP2016-87302A describe ultrasound diagnostic apparatuses that operate in a Doppler mode. JP2009-5737A, JP1995-16227A (JP-H07-16227A), and JP2004-532711A describe wall filters.

**SUMMARY OF THE INVENTION**

As described above, in the Doppler measurement, the greater the number of times the ultrasound pulses are transmitted, the greater the number of reception Doppler signals is. This allows for steeper filter characteristics, thereby enhancing the effect of suppressing unnecessary components such as clutter. However, the greater the number of times the ultrasound pulses are transmitted, the lower the frame rate is, thereby reducing the number of ultrasound images displayed per certain time.

An object of the present disclosure is to reduce the number of times ultrasound pulses are transmitted in Doppler measurement while suppressing unnecessary components included in information obtained by the Doppler measurement.

According to the present disclosure, there is provided an ultrasound diagnostic apparatus comprising: a transmission unit that transmits ultrasound pulses N times through an ultrasound probe, where N is an integer of 2 or more; a reception unit that receives reflected waves generated N times in a measurement target object through the ultrasound probe; and an information processing unit that generates N reception Doppler signals from N reception pulse signals output from the reception unit in response to the reflected waves generated N times in the measurement target object and that executes processing on each of the reception Doppler signals, in which the information processing unit includes a filter that performs high-pass filter processing on the N reception Doppler signals, a Doppler measurement section that generates Doppler measurement information of the measurement target object based on the N reception Doppler signals that have been subjected to the high-pass filter processing, and a machine learning model that is constructed based on training data, under a condition that J and K are integers of 2 or more, with J being greater than K, the training data includes training information that is at least one of reception characteristic information, which is derived from each of the reception Doppler signals in a case in which N is set to K, or the Doppler measurement information, which is generated by the Doppler measurement section in a case in which N is set to K, and target information that is at least one of the reception characteristic information, which is derived from each of the reception Doppler signals in a case in which N is set to J, or the Doppler measurement information, which is generated by the Doppler measurement section in a case in which N is set to J, for the same measurement target object as that in a case in which N is set to K, and the machine learning model generates the Doppler measurement information based on input information that is at least one of the reception characteristic information, which is derived from each of the reception Doppler signals in a case in which N is set to K, or the Doppler measurement information, which is generated by the Doppler measurement section in a case in which N is set to K, for a measurement target object in a subject.

In one embodiment, the reception characteristic information includes at least one of the N reception Doppler signals before the high-pass filter processing or the N reception Doppler signals after the high-pass filter processing.

In one embodiment, the Doppler measurement information includes at least one of autocorrelation values of the N reception Doppler signals after the high-pass filter processing, a velocity of the measurement target object obtained from the N reception Doppler signals after the high-pass filter processing, a Doppler frequency variation degree for the N reception Doppler signals after the high-pass filter processing, or a value indicating a magnitude of the N reception Doppler signals after the high-pass filter processing.

In one embodiment, the transmission unit transmits an ultrasound wave for B-mode image generation through the ultrasound probe, the reception unit receives a reflected wave for B-mode image generation generated in the measurement target object through the ultrasound probe, the information processing unit further includes a B-mode image generation section that generates B-mode image data based on a B-mode image reception signal output from the reception unit in response to the reflected wave for B-mode image generation, and the B-mode image reception signal output from the reception unit is utilized as any of the N reception pulse signals output from the reception unit in response to the reflected wave for B-mode image generation.

In one embodiment, the transmission unit forms a plurality of transmission bands at different positions or in different directions for the ultrasound pulses, for two adjacent transmission bands, the ultrasound pulses are alternately transmitted such that the ultrasound pulses are transmitted in one transmission band while no ultrasound pulses are transmitted in the other transmission band, for two adjacent transmission band pairs, each including two adjacent transmission bands, with a series of ultrasound pulses alternately transmitted in two transmission bands included in one transmission band pair as an ultrasound pulse group, the ultrasound pulse groups are alternately transmitted such that the ultrasound pulse group is transmitted in one transmission band pair while no ultrasound pulse group is transmitted in the other transmission band pair, and each time K ultrasound pulses are transmitted in each transmission band, the machine learning model generates the Doppler measurement information for one frame for each transmission band.

In one embodiment, while the Doppler measurement information for one frame is being generated, an operation of alternately transmitting the ultrasound pulse groups is performed a plurality of times for the two adjacent transmission band pairs.

According to the present disclosure, it is possible to reduce the number of times the ultrasound pulses are transmitted in the Doppler measurement while suppressing unnecessary components included in the information obtained by the Doppler measurement.

## BRIEF DESCRIPTION OF THE DRAWINGS

Fig. 1 is a diagram showing a configuration of an ultrasound diagnostic apparatus.
Fig. 2 is a diagram showing an operation in which training information is input to a machine learning section.
Fig. 3 is a diagram showing an operation in which target information is input to the machine learning section.
Fig. 4 is a diagram showing a model application operation.
Fig. 5 is a diagram showing an example of an ultrasound pulse transmitted from an ultrasound probe in a normal operation.
Fig. 6 is a diagram showing an example of an ultrasound pulse transmitted from the ultrasound probe in the model application operation.
Fig. 7 is a diagram showing the model application operation.

## DESCRIPTION OF THE PREFERRED EMBODIMENTS

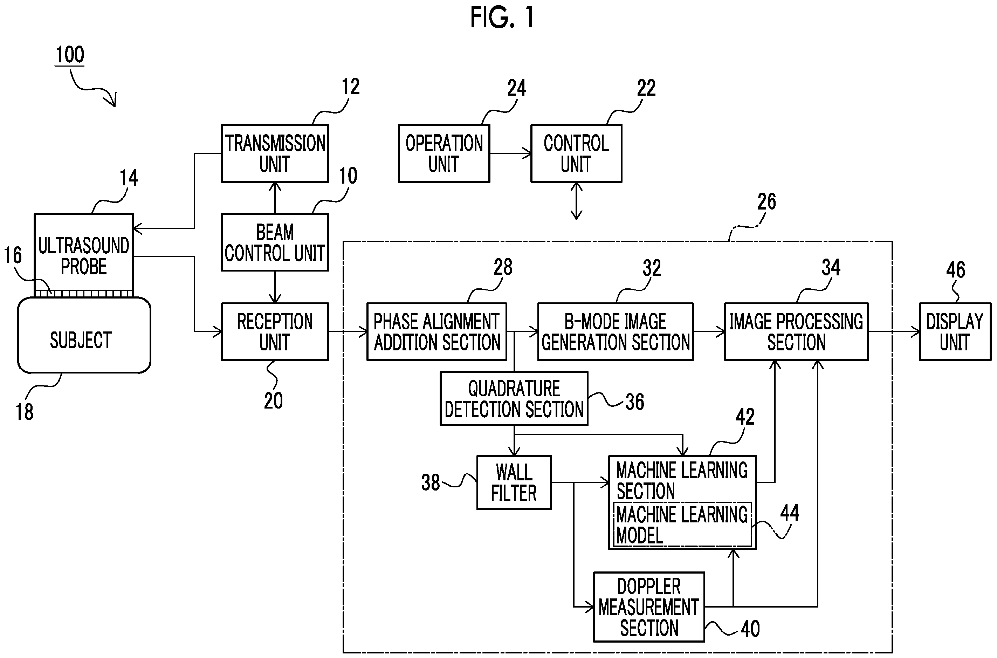

Each of embodiments of the present disclosure will be described with reference to each drawing. The same components shown in a plurality of drawings are assigned the same reference numerals, and the description thereof will not be repeated. Fig. 1 shows a configuration of an ultrasound diagnostic apparatus 100 according to the embodiment of the present disclosure. The ultrasound diagnostic apparatus 100 comprises a beam control unit 10, a transmission unit 12, an ultrasound probe 14, a reception unit 20, a control unit 22, an operation unit 24, an information processing unit 26, and a display unit 46.

The information processing unit 26 comprises a phase alignment addition section 28, a B-mode image generation section 32, an image processing section 34, a quadrature detection section 36, a wall filter 38, a Doppler measurement section 40, and a machine learning section 42. The information processing unit 26 may be configured with one or a plurality of processors that execute programs to implement respective functions of the components (the phase alignment addition section 28, the B-mode image generation section 32, the image processing section 34, the quadrature detection section 36, the wall filter 38, the Doppler measurement section 40, and the machine learning section 42). The control unit 22 performs overall control of the ultrasound diagnostic apparatus 100. The operation unit 24 includes a keyboard, a mouse, a lever, a button, a voice recognition device, and the like and outputs information related to an operation of a user to the control unit 22. The control unit 22 controls the ultrasound diagnostic apparatus 100 in response to an operation on the operation unit 24.

The operation unit 24 may constitute a user interface for operating the ultrasound diagnostic apparatus 100 together with the display unit 46. For example, the operation unit 24 may include a touch panel on a display screen of the display unit 46. In addition, the operation unit 24 may have a function of operating a keyboard, a lever, a button, and the like that are virtually configured on the display screen.

The outline of an operation of the ultrasound diagnostic apparatus 100 will be described. The ultrasound diagnostic apparatus 100 transmits ultrasound waves from the ultrasound probe 14 to a subject 18 and receives ultrasound waves reflected in the subject 18, that is, reflected waves generated in the subject 18 with the ultrasound probe 14. The ultrasound diagnostic apparatus 100 executes each measurement in a B-mode and a Doppler mode.

In the operation of the B-mode, the ultrasound diagnostic apparatus 100 generates B-mode image data based on the reflected waves received from the subject 18 and displays a B-mode image on the display unit 46. In the operation of the Doppler mode, the ultrasound diagnostic apparatus 100 generates B-mode color Doppler image data that indicates an image in which a color corresponding to a blood flow velocity is applied to a B-mode image, and displays the B-mode color Doppler image on the display unit 46.

The specific configuration of the ultrasound diagnostic apparatus 100 and the specific processing executed by the ultrasound diagnostic apparatus 100 will be described. The ultrasound probe 14 is in contact with a surface of the subject 18. The ultrasound probe 14 comprises a plurality of transducer elements 16. The transmission unit 12 outputs a transmission signal to each transducer element 16 of the ultrasound probe 14 based on the control of the beam control unit 10. Consequently, ultrasound waves are transmitted from the ultrasound probe 14. The beam control unit 10 may form a transmission beam in the ultrasound probe 14 by controlling the transmission unit 12 and scan the subject 18 with the transmission beam. That is, the transmission unit 12 may form the transmission beam in the ultrasound probe 14 by adjusting a delay time or level of each transmission signal in accordance with the control of the beam control unit 10 and scan the subject 18 with the transmission beam. In addition, the transmission unit 12 may form a transmission band composed of a plurality of transmission beams in the ultrasound probe 14 by adjusting the delay time or level of each transmission signal in accordance with the control of the beam control unit 10.

In a case in which the reflected wave generated in the subject 18 is received by each transducer element 16 of the ultrasound probe 14, each transducer element 16 outputs a reception signal corresponding to the received ultrasound wave to the reception unit 20. The reception unit 20 performs processing, such as amplification and frequency band limitation, on the reception signal output from each transducer element 16 in accordance with the control of the beam control unit 10, and outputs the processed reception signal to the information processing unit 26. The information processing unit 26 performs processing executed by each component (the phase alignment addition section 28, the B-mode image generation section 32, the image processing section 34, the quadrature detection section 36, the wall filter 38, the Doppler measurement section 40, and the machine learning section 42) on each reception signal.

The phase alignment addition section 28 generates a phase-aligned reception signal by performing phase alignment addition on the plurality of reception signals that are output from the reception unit 20 to the plurality of transducer elements 16. Consequently, the phase-aligned reception signal obtained by adjusting and adding phases such that the reception signals based on the ultrasound waves received from a specific direction strengthen each other is generated, and a reception beam is formed in the specific direction. The phase alignment addition section 28 outputs the phase-aligned reception signal to the B-mode image generation section 32 during the operation in the B-mode. Additionally, the phase alignment addition section 28 outputs the phase-aligned reception signal to the quadrature detection section 36 during the operation in the Doppler mode.

The operation in the B-mode will be described. The phase alignment addition section 28 generates each phase-aligned reception signal based on the ultrasound wave received from each direction of the reception beam with which the subject 18 is scanned, and outputs the phase-aligned reception signal to the B-mode image generation section 32 as a B-mode image reception signal. The B-mode image generation section 32 generates B-mode image data based on the B-mode image reception signal obtained in each reception beam direction and outputs the B-mode image data to the image processing section 34. The B-mode image data based on a single scan with the transmission beam and the reception beam (hereinafter, referred to as a transmission and reception beam) is image data for one frame and corresponds to one B-mode image.

The beam control unit 10, the transmission unit 12, the ultrasound probe 14, the reception unit 20, the phase alignment addition section 28, and the B-mode image generation section 32 generate the B-mode image data one after another while performing repeated scans with the transmission and reception beams, and output each B-mode image data to the image processing section 34. The image processing section 34 generates a video signal for displaying the B-mode image based on the B-mode image data and outputs the video signal to the display unit 46. The display unit 46 displays the B-mode image based on the video signal.

The B-mode may be a phase inversion B-mode. In the phase inversion B-mode, the ultrasound pulses are transmitted in the same direction twice with a phase difference of 180°. The B-mode image generation section 32 generates a harmonic reception signal by adding two phase-aligned reception signals based on the reflected waves received twice in response to the ultrasound pulses transmitted twice and generates phase inversion B-mode image data based on the harmonic reception signal. The harmonic reception signal indicates harmonic components generated in the two phase-aligned reception signals due to the Doppler frequency being non-zero. The phase inversion B-mode image data indicates an image of a moving measurement target object, such as blood.

The operation in the Doppler mode will be described. The operation in the Doppler mode according to the present embodiment includes a basic operation, a learning operation, and a model application operation. In the basic operation, the Doppler measurement section 40 generates the Doppler measurement information based on the reception Doppler signal obtained by quadrature detection of the phase-aligned reception signal.

The Doppler measurement information may include color Doppler data. The color Doppler data is data that indicates a blood flow velocity by using colors on a B-mode image of a region scanned with the transmission and reception beam. The color Doppler data may be, for example, data in which a region in which blood flows in a direction away from the ultrasound probe 14 is colored blue, a region in which blood flows in a direction close to the ultrasound probe 14 is colored red, and the brightness is increased in a region with a higher blood flow velocity.

The Doppler measurement information generated by the Doppler measurement section 40 may include various types of information related to the blood flow velocity in the region scanned with the transmission and reception beam, in addition to the color Doppler data. The information other than the color Doppler data included in the Doppler measurement information will be described below.

The learning operation is an operation of generating training data for machine learning and constructing a machine learning model 44 in the machine learning section 42 through machine learning. The training data is generated through the basic operation. In the model application operation, the Doppler measurement information is generated by the machine learning model 44 constructed by the machine learning section 42 through machine learning.

The basic operation will be described. In the basic operation, the ultrasound pulses are transmitted and received N times for one transmission and reception beam. Here, N is an integer of 2 or more. N reception pulse signals are output as N phase-aligned reception signals to the quadrature detection section 36 from the phase alignment addition section 28. The quadrature detection section 36 generates N reception Doppler signals by performing quadrature detection on the N reception pulse signals with a local signal having a frequency of the transmitted ultrasound pulse. The N reception Doppler signals are output from the quadrature detection section 36 to the wall filter 38. The wall filter 38 performs high-pass filter processing on the N reception Doppler signals and outputs the N reception Doppler signals after the high-pass filter processing to the Doppler measurement section 40.

The Doppler measurement section 40 generates the color Doppler data as one piece of information included in the Doppler measurement information, based on the N reception Doppler signals acquired for each of a plurality of transmission and reception beams having different positions or directions.

The Doppler measurement section 40 obtains a blood flow velocity $v(r)$ in a depth direction of the transmission and reception beam based on the N reception Doppler signals, for example, in accordance with autocorrelation processing described in JP2016-87302A. Here, $v(r)$ indicates a blood flow velocity at a position of a depth r. The Doppler measurement section 40 generates the color Doppler data based on the blood flow velocity $v(r)$ in the depth direction at each depth r obtained for each of the plurality of transmission and reception beams having different positions or directions and outputs the color Doppler data to the image processing section 34. Hereinafter, specific processing of obtaining the blood flow velocity $v(r)$ will be described.

A reception Doppler signal acquired at the q-th (q = 1 to N) position among the first to N-th reception Doppler signals is represented by the following (Equation 1).

Equation 1

$$z(r, q) = I(r, q) + j \cdot Q(r, q)$$

r is a coordinate value in the depth direction. $I(r, q)$ indicates an in-phase component of the reception Doppler signal, and $Q(r, q)$ indicates a quadrature component of the reception Doppler signal. j is an imaginary unit. The Doppler measurement section 40 obtains an autocorrelation value $A(r)$ for N reception Doppler signals $z(r,1)$, $z(r,2)$, ..., $z(r,N)$ in accordance with (Equation 2).

Equation 2

$$A(r) = \sum_{q=1}^{J-1} z(r,q) \cdot z(r,q+1)^*$$

Here, the superscript "*" indicates a complex conjugate number. The Doppler measurement section 40 obtains the blood flow velocity v(r) in accordance with (Equation 3) by using the real part Re[A(r)] and the imaginary part Im[A(r)] of the autocorrelation value A(r).

Equation 3

$$v(r) = \frac{c}{2f_0} \cdot \frac{1}{2\pi T} arctan\left[\frac{Im[A(r)]}{Re[A(r)]}\right]$$

c is a propagation velocity of the ultrasound pulse propagating in the subject 18. $f_0$ is a frequency of the ultrasound pulse transmitted from the ultrasound probe 14. T is a time interval for transmitting N ultrasound pulses. A right-side portion with respect to $c/(2f_0)$ on the right side of (Equation 3) represents the Doppler frequency. The blood flow velocity v(r) represents a component of the blood flow velocity in the depth direction at the depth r. In a case in which an angle φ formed between the direction of the blood flow and the depth direction is known, v(r)/cosφ is the blood flow velocity.

In a case in which an absolute value of the blood flow velocity (for example, a value in an MKSA unit system) is not required to be obtained, such as in a case in which the blood flow velocity distribution is measured, the blood flow velocity may be a value obtained by multiplying a value obtained in (Equation 3) by any constant. In addition, instead of the blood flow velocity, a Doppler shift frequency measurement value, which is a right-side portion with respect to $c/(2f_0)$ on the right side of (Equation 3), may be used.

The image processing section 34 generates data that indicates a B-mode color Doppler image in which a color corresponding to the blood flow velocity is applied to the B-mode image, based on the B-mode image data and the color Doppler data. The image processing section 34 generates a video signal based on the B-mode color Doppler image data and outputs the video signal to the display unit 46. The display unit 46 displays the B-mode color Doppler image based on the video signal.

The Doppler measurement section 40 may use the autocorrelation value A(r) at each depth r obtained for each of the plurality of transmission and reception beams having different positions or directions as one piece of the Doppler measurement information.

In addition, the Doppler measurement section 40 may use the velocity v(r) at each depth r obtained for each of the plurality of transmission and reception beams having different positions or directions as one piece of the Doppler measurement information.

The Doppler measurement section 40 may generate blood flow power P(r) at each depth r obtained for each of the transmission and reception beams having different positions or directions as one piece of the Doppler measurement information. The blood flow power P(r) is represented by the magnitude of the reception Doppler signal indicated in (Equation 1). In order for the Doppler measurement section 40 to obtain the blood flow power P(r), an appropriate filter such as a singular value decomposition (SVD) filter may be used as the wall filter 38.

Additionally, the Doppler measurement section 40 may generate a degree of variation in the Doppler frequency of the reception Doppler signals acquired N times for one transmission and reception beam (hereinafter, referred to as a Doppler frequency variation degree) as one piece of information included in the Doppler measurement information.

In a case in which the blood flow velocity v(r) is obtained in accordance with (Equation 1) to (Equation 3), the Doppler frequency variation degree may be obtained based on N-1 adjacent time Doppler frequencies obtained in accordance with (Equation 4).

That is, the Doppler measurement section 40 obtains the adjacent time Doppler frequency fa(r, q) for each of q = 1 to N - 1 in accordance with (Equation 4) by using the real part and the imaginary part of z(r, q) · z(r, q + 1)* added and summed on the right side of (Equation 2).

Equation 4

$$fa(r,q) = \frac{1}{2\pi T} arctan \left[ \frac{Im[z(r,q) \cdot z(r,q+1)^*]}{Re[z(r,q) \cdot z(r,q+1)^*]} \right]$$

The Doppler measurement section 40 obtains the Doppler frequency variation degree for N-1 adjacent time Doppler frequencies fa(r, 1), fa(r, 2), .. , fa(r, N-1) and outputs the Doppler frequency variation degree to the image processing section 34. The Doppler frequency variation degree may be a value indicating a variation such as a variance or a standard deviation for the N-1 adjacent time Doppler frequencies.

The image processing section 34 may adjust the color to be applied to the B-mode image according to the Doppler frequency variation degree. For example, in a case in which a region in which blood flows in a direction away from the ultrasound probe 14 is colored blue and a region in which blood flows in a direction close to the ultrasound probe 14 is colored red, the greater the Doppler frequency variation degree is, the closer the color may be to purple or white.

As described above, the Doppler measurement information generated by the Doppler measurement section 40 includes, in addition to the color Doppler data, the autocorrelation value A(r), the blood flow velocity v(r), the blood flow power P(r), the Doppler frequency variation degree, and the like obtained for each of the transmission and reception beams having different positions or directions. The Doppler measurement information may be information including at least one of the autocorrelation value A(r), the blood flow velocity v(r), the blood flow power P(r), or the Doppler frequency variation degree.

In this way, in the basic operation, the Doppler measurement section 40 generates the Doppler measurement information based on the N reception Doppler signals that have been subjected to the high-pass filter processing by the wall filter 38. The reception Doppler signal output from the quadrature detection section 36 includes low-frequency noise based on the motion of the tissue in the subject 18, which causes unnecessary components included in the Doppler measurement information. For example, this causes noise referred to as clutter in the color Doppler data. Therefore, the unnecessary components included in the Doppler measurement information are suppressed by the wall filter 38.

Next, the learning operation will be described. In the learning operation, in a case in which the ultrasound pulses are transmitted and received K times for each of the transmission and reception beams, the following processing is executed. Here, K is an integer of 2 or more and smaller than N. As the ultrasound pulses are transmitted and received K times for each of the transmission and reception beams, K reception Doppler signals are output from the quadrature detection section 36 to the machine learning section 42 and the wall filter 38.

The wall filter 38 performs high-pass filter processing on the K reception Doppler signals and outputs the K reception Doppler signals after the high-pass filter processing to the Doppler measurement section 40 and the machine learning section 42. The Doppler measurement section 40 generates the Doppler measurement information based on the reception Doppler signals acquired K times for each of the plurality of transmission and reception beams having different positions or directions and outputs the Doppler measurement information to the machine learning section 42.

In this way, in a case in which the ultrasound pulses are transmitted and received K times for each of the transmission and reception beams (hereinafter, may be referred to as K transmissions and receptions), the following information is input to the machine learning section 42 as the training information. That is, the reception Doppler signal before the high-pass filter processing is performed by the wall filter 38, the reception Doppler signal after the high-pass filter processing is performed by the wall filter 38, and the Doppler measurement information generated by the Doppler measurement section 40 are input to the machine learning section 42.

The training information may include at least one of the reception Doppler signal before the high-pass filter processing is performed by the wall filter 38, the reception Doppler signal after the high-pass filter processing is performed by the wall filter 38, or the Doppler measurement information generated by the Doppler measurement section 40. The Doppler measurement information may be at least one of a plurality of types of information included in the Doppler measurement information.

Next, in a case in which the ultrasound pulses are transmitted and received J times for each of the transmission and reception beams (hereinafter, may be referred to as J transmissions and receptions), the following information is input to the machine learning section 42 as the target information. Here, J is an integer of 2 or more and greater than K. In a case of the J transmissions and receptions, a tap coefficient of the wall filter 38 is greater than that in a case of the K transmissions and receptions. Therefore, in the case of the J transmissions and receptions, as compared with the case of the K transmissions and receptions, low-pass filter characteristics are steeper, resulting in more pronounced characteristics in which the attenuation amount in the high-frequency range is smaller and the attenuation amount in the low-frequency range is larger.

The reception Doppler signal before the high-pass filter processing is performed by the wall filter 38, the reception Doppler signal after the high-pass filter processing is performed by the wall filter 38, and the Doppler measurement information generated by the Doppler measurement section 40 are input to the machine learning section 42. However, a region (measurement target object) in which the ultrasound pulses are transmitted and received in the case of the J transmissions and receptions is the same as a region (measurement target object) in which the ultrasound pulses are transmitted and received in the case of the K transmissions and receptions. The Doppler measurement information as the target information may be at least one of the plurality of types of information included in the Doppler measurement information.

In the learning operation, the K transmissions and receptions and the J transmissions and receptions are performed for blood flows in a plurality of different measurement target objects, for example, in a plurality of different observation sites. The machine learning section 42 acquires, as training data, a set of the training information generated through the K transmissions and receptions and the target information generated through the J transmissions and receptions for a plurality of different subjects 18 or a plurality of different observation sites and constructs the machine learning model 44. For at least one of the plurality of different measurement target objects, at least the Doppler measurement information is included as the target information. The learning operation may be performed by using a machine learning algorithm such as CNN, AdaBoost, or SVN. The machine learning algorithm may include a deep learning algorithm.

The constructed machine learning model 44 is a model that generates Doppler measurement information equivalent to the case of the J transmissions and receptions, in response to the input of the following input information in a case in which the ultrasound pulses are transmitted and received K times for each of the transmission and reception beams. That is, the machine learning model 44 is a model that predicts the Doppler measurement information that may be obtained assuming that the ultrasound pulses are transmitted and received J times, in a case in which the ultrasound pulses are transmitted and received K times.

The input information may be at least one of the reception Doppler signal before the high-pass filter processing is performed by the wall filter 38, the reception Doppler signal after the high-pass filter processing is performed by the wall filter 38, or the Doppler measurement information generated by the Doppler measurement section 40. The Doppler measurement information may be at least one of the plurality of types of information included in the Doppler measurement information.

Next, the model application operation will be described. In the model application operation, the observation site of the subject 18 is scanned with the ultrasound beam, and the ultrasound pulses are transmitted and received for a plurality of transmission and reception beams having different positions or directions. For each of the transmission and reception beams, the ultrasound pulses are transmitted and received K times, and the K reception Doppler signals are output from the quadrature detection section 36 to the wall filter 38 and the machine learning model 44.

The wall filter 38 performs high-pass filter processing on the K reception Doppler signals and outputs the K reception Doppler signals after the high-pass filter processing to the Doppler measurement section 40 and the machine learning model 44. The Doppler measurement section 40 generates the Doppler measurement information based on the reception Doppler signals acquired K times for each of the plurality of transmission and reception beams having different positions or directions and outputs the Doppler measurement information to the machine learning model 44.

As a result, the input information is input to the machine learning model 44 in a case in which the ultrasound pulses are transmitted and received K times for each of the transmission and reception beams, and the machine learning model 44 generates the Doppler measurement information equivalent to the case of the J transmissions and receptions for the input information.

In a case in which the Doppler measurement information includes the color Doppler data, the machine learning model 44 outputs the color Doppler data to the image processing section 34. The image processing section 34 generates data that indicates the B-mode color Doppler image based on the B-mode image data and the color Doppler data. The image processing section 34 generates the video signal for displaying the B-mode color Doppler image based on the B-mode color Doppler image data and outputs the video signal to the display unit 46. The display unit 46 displays the B-mode color Doppler image based on the video signal.

The image processing section 34 may generate composite image data in which an image based on the B-mode color Doppler image data obtained through the normal operation and an image based on the B-mode color Doppler image data obtained through the model application operation are displayed side by side. In addition, the image processing section 34 may generate composite image data that indicates the image based on the B-mode color Doppler image data obtained through the normal operation and the image based on the B-mode color Doppler image data obtained through the model application operation such that one image is visible through the other image. The image processing section 34 generates a video signal for displaying a composite image based on the composite image data and outputs the video signal to the display unit 46. The display unit 46 displays the composite image based on the video signal.

In this way, the ultrasound diagnostic apparatus 100 according to the present embodiment comprises the transmission unit 12, the reception unit 20, and the information processing unit 26. The transmission unit 12 transmits the ultrasound pulses N times through the ultrasound probe 14. The reception unit 20 receives reflected waves generated N times in the measurement target object through the ultrasound probe 14. The information processing unit 26 generates the N reception Doppler signals from the N reception pulse signals output from the reception unit 20 through the quadrature detection section 36 in response to the reflected waves generated N times and executes processing on the N reception Doppler signals. The measurement target object in the present embodiment is an observation site in which blood flows in the subject 18.

The information processing unit 26 includes the wall filter 38 (filter) that performs the high-pass filter processing on the N reception Doppler signals, the Doppler measurement section 40 that generates the Doppler measurement information of the measurement target object based on the N reception Doppler signals that have been subjected to the high-pass filter processing, and the machine learning model 44 that is constructed based on the training data.

The training data includes the training information and the target information. The training information is at least one of the reception characteristic information derived from each of the reception Doppler signals in a case in which N is set to K, or the Doppler measurement information generated by the Doppler measurement section 40 in a case in which N is set to K. Here, the reception characteristic information includes at least one of the reception Doppler signal before the high-pass filter processing is performed or the reception Doppler signal after the high-pass filter processing is performed.

The target information is at least one of the reception characteristic information derived from each of the reception Doppler signals in a case in which N is set to J, or the Doppler measurement information generated by the Doppler measurement section 40 in a case in which N is set to J, for the same measurement target object as that in a case in which N is set to K. Here, J and K are integers of 2 or more, and J is greater than K.

The machine learning model 44 generates the Doppler measurement information based on the input information. The input information is at least one of the reception characteristic information derived from each of the reception Doppler signals in a case in which N is set to K, or the Doppler measurement information generated by the Doppler measurement section 40 in a case in which N is set to K, for the measurement target object in the subject 18.

As described above, in the case of the J transmissions and receptions in which N is set to J, the tap coefficient of the wall filter 38 is greater than that in the case of the K transmissions and receptions in which N is set to K. Therefore, in the case of the J transmissions and receptions, as compared with the case of the K transmissions and receptions, the low-pass filter characteristics are steeper, resulting in more pronounced characteristics in which the attenuation amount in the high-frequency range is smaller and the attenuation amount in the low-frequency range is larger. Accordingly, the unnecessary components included in the Doppler measurement information are significantly suppressed in the J transmissions and receptions as compared with the K transmissions and receptions. For the color Doppler data included in the Doppler measurement information, the J transmissions and receptions have a greater effect in suppressing clutter as compared with the K transmissions and receptions.

With the ultrasound diagnostic apparatus 100 according to the present embodiment, by applying the machine learning model 44, the Doppler measurement information equivalent to the case of the J transmissions and receptions can be obtained even in the case of the K transmissions and receptions. Consequently, even in a case in which the number of transmissions of the ultrasound pulses is relatively small, the unnecessary components included in the Doppler measurement information are suppressed by the wall filter 38. In displaying the B-mode color Doppler image, clutter is suppressed by the wall filter 38 even in a case in which the number of transmissions of the ultrasound pulses is relatively small.

In addition, in the ultrasound diagnostic apparatus 100 according to the present embodiment, the number of transmissions of the ultrasound pulses is reduced to increase the frame rate in a case in which the color Doppler data is generated, while maintaining the effect of suppressing the unnecessary components included in the Doppler measurement information.

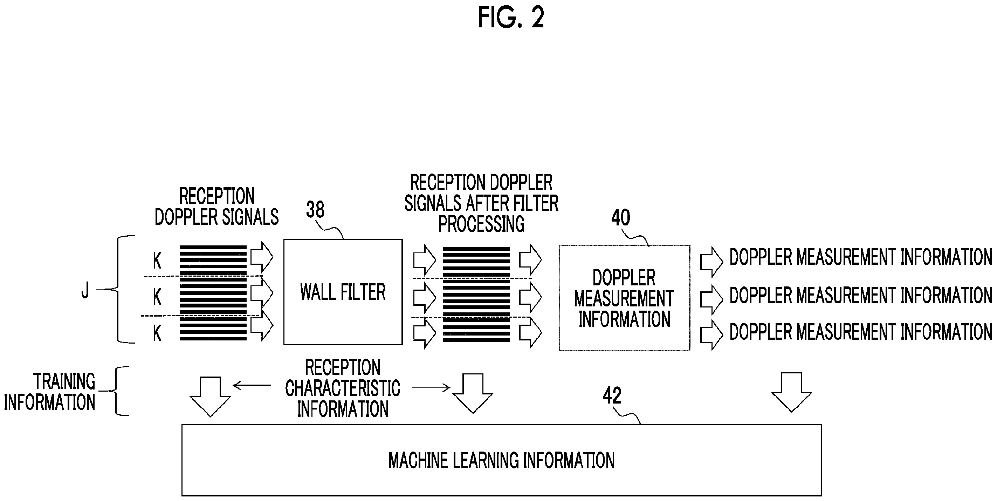

Fig. 2 shows an operation in which the training information is input to the machine learning section 42. Fig. 2 shows that J = 3K is established, and the training information is input to the machine learning section 42 three times for the J transmissions and receptions of a series of ultrasound pulses. That is, as J ultrasound pulses are transmitted from the ultrasound probe 14, and J reflected waves are received by the ultrasound probe 14, J reception Doppler signals are input to the wall filter 38 and the machine learning section 42.

The wall filter 38, the Doppler measurement section 40, and the machine learning section 42 input the training information to the machine learning section 42 for each set of K reception Doppler signals. That is, the wall filter 38 outputs the K reception Doppler signals after the high-pass filter processing to the Doppler measurement section 40 and the machine learning section 42 each time the K reception Doppler signals are input. The Doppler measurement section 40 outputs the Doppler measurement information to the machine learning section 42 each time the K reception Doppler signals after the high-pass filter processing are input. In this way, the K reception Doppler signals, the K reception Doppler signals before the high-pass filter processing, the K reception Doppler signals after the high-pass filter processing, and the Doppler measurement information generated according to the K reception Doppler signals are input, so that the training information corresponding to the K transmissions and receptions is input to the machine learning section 42 three times.

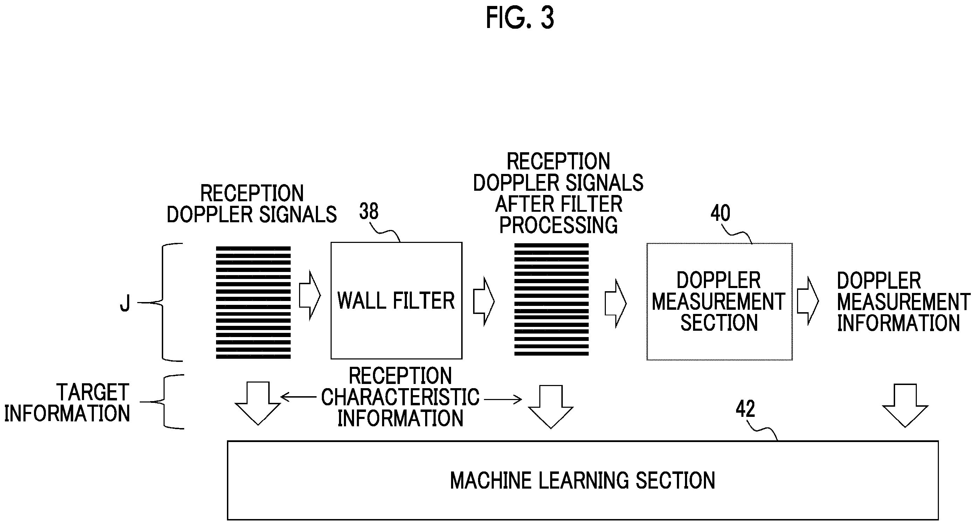

Fig. 3 shows an operation in which the target information is input to the machine learning section 42. Fig. 3 shows that the target information is input to the machine learning section 42 once for the J transmissions and receptions of the series of ultrasound pulses. That is, as the J ultrasound pulses are transmitted from the ultrasound probe 14, and the J reflected waves are received by the ultrasound probe 14, the J reception Doppler signals are input to the wall filter 38 and the machine learning section 42. The J reception Doppler signals shown in Fig. 3 may be the same as the J reception Doppler signals shown in Fig. 2. That is, the J reception Doppler signals obtained through the J = 3K transmissions and receptions in the operation in which the training information is input to the machine learning section 42 may be used in the operation in which the target information is input to the machine learning section 42.

The wall filter 38, the Doppler measurement section 40, and the machine learning section 42 input the target information based on the J reception Doppler signals to the machine learning section 42. That is, the wall filter 38 outputs the J reception Doppler signals after the high-pass filter processing to the Doppler measurement section 40 and the machine learning section 42 based on the J reception Doppler signals. The Doppler measurement section 40 outputs the Doppler measurement information to the machine learning section 42 in response to the input of the J reception Doppler signals after the high-pass filter processing. In this way, the J reception Doppler signals, the J reception Doppler signals before the high-pass filter processing, the J reception Doppler signals after the high-pass filter processing, and the Doppler measurement information generated according to the J reception Doppler signals are input to the machine learning section 42 as the target information corresponding to the J transmissions and receptions.

The operations shown in Figs. 2 and 3 are performed for the plurality of different subjects 18 or the plurality of different observation sites, and the machine learning section 42 constructs the machine learning model 44 based on the training information input a plurality of times and the target information input a plurality of times.

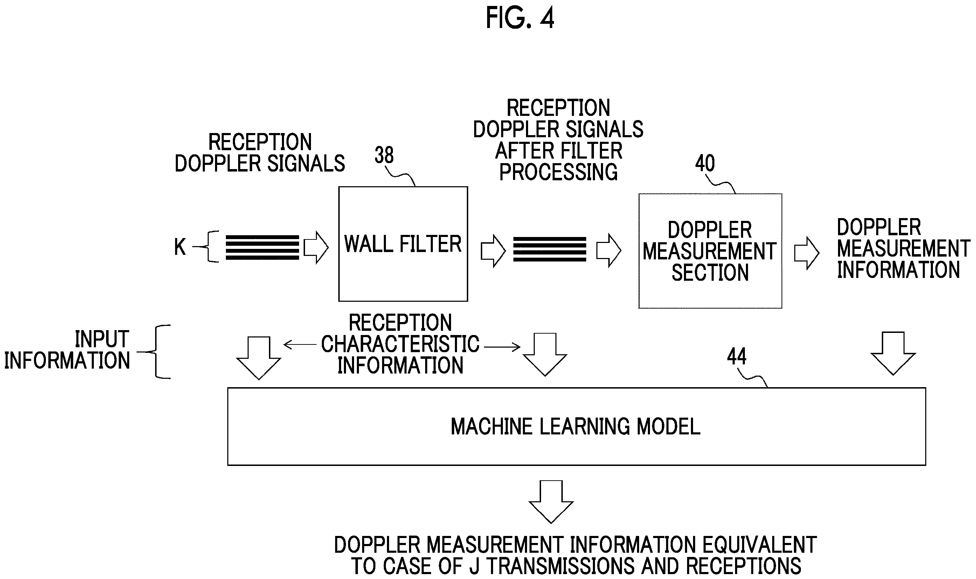

Fig. 4 shows the model application operation. Fig. 4 shows that the K reception Doppler signals before the high-pass filter processing is performed, the K reception Doppler signals after the high-pass filter processing is performed, and the Doppler measurement information are input to the machine learning model 44 once for the K transmissions and receptions of the series of ultrasound pulses. That is, as the K ultrasound pulses are transmitted from the ultrasound probe 14, and the K reflected waves are received by the ultrasound probe 14, the K reception Doppler signals are input to the wall filter 38 and the machine learning model 44. The wall filter 38 outputs the K reception Doppler signals after the high-pass filter processing to the Doppler measurement section 40 and the machine learning model 44 based on the K reception Doppler signals. The Doppler measurement section 40 outputs the Doppler measurement information to the machine learning model 44 in response to the input of the K reception Doppler signals after the high-pass filter processing. In this way, the K reception Doppler signals before the high-pass filter processing, the K reception Doppler signals after the high-pass filter processing, and the Doppler measurement information generated according to the K reception Doppler signals are input to the machine learning model 44 as the input information, and the machine learning model 44 generates the Doppler measurement information equivalent to the case of the J transmissions and receptions based on the input information.

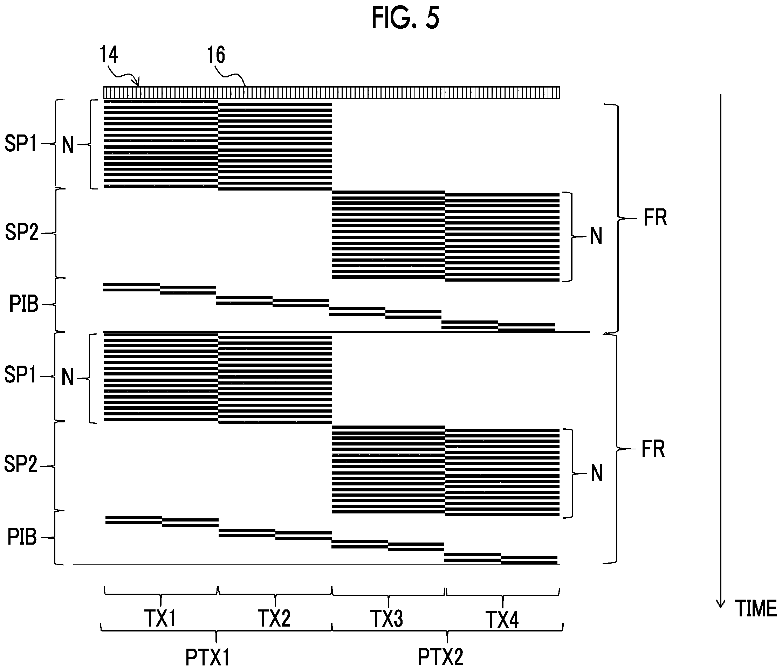

Fig. 5 shows an example of the ultrasound pulse transmitted from the ultrasound probe 14 in the normal operation. The plurality of transducer elements 16 provided in the ultrasound probe 14 are arranged in a left-right direction of Fig. 5. The vertical axis direction represents time. In the embodiment shown in Fig. 5, four transmission bands TX1 to TX4 are formed at different lateral positions by the plurality of transducer elements 16 arranged in the left-right direction. Each of the transmission bands TX1 to TX4 is formed by a plurality of transmission beams. In each of the transmission bands TX1 to TX4, the N ultrasound pulses are transmitted, and N reflected waves generated by being reflected in the subject 18 are received. A series of N ultrasound pulses are used as an ultrasound pulse group, and the ultrasound pulse group is transmitted at a predetermined time interval.

In adjacent transmission bands TX1 and TX2, ultrasound pulses are alternately transmitted, and the N ultrasound pulses are transmitted in each of the transmission bands TX1 and TX2. Similarly, in adjacent transmission bands TX3 and TX4, ultrasound pulses are alternately transmitted, and the N ultrasound pulses are transmitted in each of the transmission bands TX3 and TX4.

After an ultrasound pulse group SP1 is transmitted in the adjacent transmission bands TX1 and TX2, an ultrasound pulse group SP2 is transmitted in the adjacent transmission bands TX3 and TX4. The transmission of the ultrasound pulse group SP1 in the adjacent transmission bands TX1 and TX2 and the transmission of the ultrasound pulse group SP2 in the adjacent transmission bands TX3 and TX4 are alternately repeated. In the following description, the adjacent transmission bands TX1 and TX2 are referred to as a transmission band pair PTX1, and the adjacent transmission bands TX3 and TX4 are referred to as a transmission band pair PTX2.

After the transmission of the ultrasound pulse group SP2 in the transmission band pair PTX2 and before the transmission of the ultrasound pulse group SP1 in the transmission band pair PTX1, in the ultrasound probe 14, ultrasound pulses for B-mode image generation are transmitted and reflected waves for B-mode image generation are received. In the embodiment shown in Fig. 5, a half-transmission band for B-mode image generation is formed with a width that is half of the width of each of the transmission bands TX1 to TX4. Two ultrasound pulses for B-mode image generation are transmitted in each of two adjacent half-transmission bands in the transmission band TX1, and the ultrasound pulses for B-mode image generation are alternately transmitted in the two adjacent half-transmission bands in the transmission band TX1. In the present embodiment, the B-mode is the phase inversion B-mode, and the phase difference between the two ultrasound pulses for B-mode image generation transmitted in each half-transmission band is 180°.

In the present embodiment, an operation of alternately transmitting the ultrasound pulses for B-mode image generation in the two half-transmission bands in each transmission band in the order of the transmission bands TX1, TX2, TX3, and TX4 is executed. In Fig. 5, the ultrasound pulse group for B-mode image generation in a case in which the ultrasound pulses for B-mode image generation are alternately transmitted in the two half-transmission bands in each of the transmission bands TX1, TX2, TX3, and TX4 is shown as an ultrasound pulse group PIB.

The ultrasound pulse group SP1 is transmitted in the transmission band pair PTX1, the ultrasound pulse group SP2 is transmitted in the transmission band pair PTX2, the ultrasound pulse group PIB is transmitted in each of the transmission bands TX1, TX2, TX3, and TX4, and the processing for one frame FR is executed by the reception processing for the reflected waves generated by each ultrasound pulse group. The processing for one frame FR corresponds to processing of generating the Doppler measurement information corresponding to one color Doppler image.

For each of the transmission bands TX1 to TX4, in the processing for one frame FR, the normal operation in a case in which the N ultrasound pulses are transmitted and the N reflected waves generated by being reflected in the subject 18 are received is executed, and the Doppler measurement section 40 generates the Doppler measurement information without relying on the machine learning model 44.

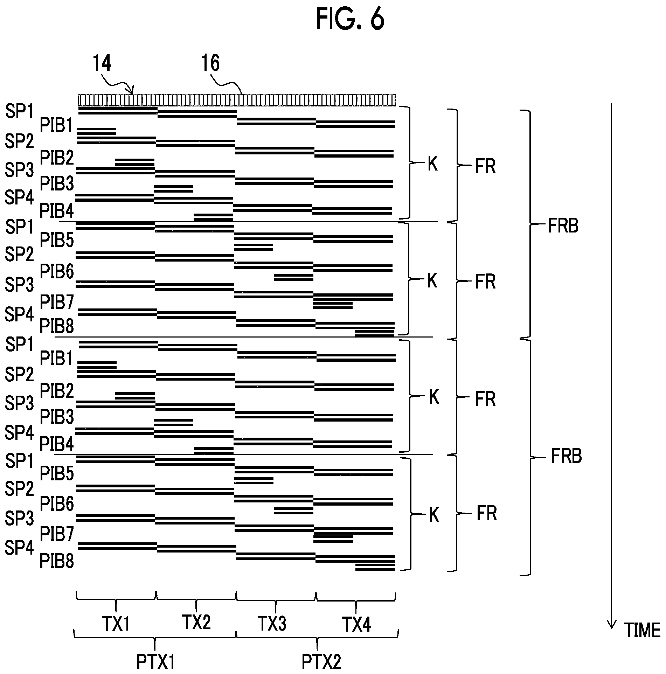

Fig. 6 shows an example of the ultrasound pulse transmitted from the ultrasound probe 14 in the model application operation. Even in the embodiment shown in Fig. 6, as in the embodiment shown in Fig. 5, four transmission bands TX1 to TX4 are formed at different lateral positions for the ultrasound pulses by the plurality of transducer elements 16 arranged in the left-right direction.

Two ultrasound pulses are transmitted in each of the adjacent transmission bands TX1 and TX2, and the ultrasound pulses are alternately transmitted in the adjacent transmission bands TX1 and TX2. Similarly, two ultrasound pulses are transmitted in each of the adjacent transmission bands TX3 and TX4, and the ultrasound pulses are alternately transmitted in the adjacent transmission bands TX3 and TX4. In this way, in each of the transmission bands TX1, TX2, TX3, and TX4, the ultrasound pulse group SP1 consisting of two ultrasound pulses is transmitted. Similarly, in each of the transmission bands TX1, TX2, TX3, and TX4, ultrasound pulse groups SP2, SP3, and SP4 each consisting of two ultrasound pulses are transmitted in order with the passage of time.

In the present embodiment, a series of transmission processing of transmitting the ultrasound pulse groups SP1 to SP4 in order with the passage of time is repeated in each of the transmission bands TX1, TX2, TX3, and TX4. In each of the transmission bands TX1, TX2, TX3, and TX4, the ultrasound pulse groups SP1 to SP4 are transmitted, so that the K ultrasound pulses are transmitted in each of the transmission bands TX1, TX2, TX3, and TX4. In the example shown in Fig. 6, K = 8.

The transmission of the ultrasound pulses for B-mode image generation, which are transmitted in conjunction with the transmission of the initial series of ultrasound pulse groups SP1 to SP4 in Fig. 6, will be described. After the transmission of the ultrasound pulse group SP1 in the transmission band TX4 and before the transmission of the ultrasound pulse group SP2 in the transmission band TX1, an ultrasound pulse group PIB1 for B-mode image generation is transmitted in the half-transmission band on the left side of the transmission band TX1. After the transmission of the ultrasound pulse group SP2 in the transmission band TX4 and before the transmission of the ultrasound pulse group SP3 in the transmission band TX1, an ultrasound pulse group PIB2 for B-mode image generation is transmitted in the half-transmission band on the right side of the transmission band TX1.

After the transmission of the ultrasound pulse group SP3 in the transmission band TX4 and before the transmission of the ultrasound pulse group SP4 in the transmission band TX1, an ultrasound pulse group PIB3 for B-mode image generation is transmitted in the half-transmission band on the left side of the transmission band TX2. After the transmission of the ultrasound pulse group SP4 in the transmission band TX4 and before the transmission of the ultrasound pulse group SP1 in the transmission band TX1, an ultrasound pulse group PIB4 for B-mode image generation is transmitted in the half-transmission band on the right side of the transmission band TX2.

The transmission of the ultrasound pulses for B-mode image generation, which are transmitted in conjunction with the transmission of the second series of ultrasound pulse groups SP1 to SP4 in Fig. 6, will be described. After the transmission of the ultrasound pulse group SP1 in the transmission band TX4 and before the transmission of the ultrasound pulse group SP2 in the transmission band TX1, an ultrasound pulse group PIB5 for B-mode image generation is transmitted in the half-transmission band on the left side of the transmission band TX3. After the transmission of the ultrasound pulse group SP2 in the transmission band TX4 and before the transmission of the ultrasound pulse group SP3 in the transmission band TX1, an ultrasound pulse group PIB6 for B-mode image generation is transmitted in the half-transmission band on the right side of the transmission band TX3. After the transmission of the ultrasound pulse group SP3 in the transmission band TX4 and before the transmission of the ultrasound pulse group SP4 in the transmission band TX1, an ultrasound pulse group PIB7 for B-mode image generation is transmitted in the half-transmission band on the left side of the transmission band TX4. After the transmission of the ultrasound pulse group SP4 in the transmission band TX4 and before the transmission of the ultrasound pulse group SP1 in the transmission band TX1, an ultrasound pulse group PIB8 for B-mode image generation is transmitted in the half-transmission band on the right side of the transmission band TX4.

Thereafter, as the odd-numbered series of ultrasound pulse groups SP1 to SP4 of Fig. 6 are transmitted, the ultrasound pulse groups PIB1 to PIB4 are transmitted at the same timings as the ultrasound pulse groups PIB1 to PIB4 transmitted in conjunction with the transmission of the initial series of ultrasound pulse groups SP1 to SP4. In addition, as the even-numbered series of ultrasound pulse groups SP1 to SP4 in Fig. 6 are transmitted, the ultrasound pulse groups PIB5 to PIB8 are transmitted at the same timings as the ultrasound pulse groups PIB5 to PIB8 transmitted in conjunction with the transmission of the second series of ultrasound pulse groups SP1 to SP4.

The ultrasound pulse groups SP1 to SP4 are transmitted in each of the transmission bands TX1 to TX4, and the processing for one frame FR of the color Doppler data is executed by the reception processing for the reflected waves generated by each ultrasound pulse group. The processing for one frame FR corresponds to processing of generating the Doppler measurement information corresponding to one color Doppler image.

In addition, the ultrasound pulse groups PIB1 to PIB8 for B-mode image generation are transmitted from the half-transmission band formed in each of the transmission bands TX1 to TX4, and the processing for one frame FRB of phase inversion B-mode data is executed by the reception processing for the reflected waves generated by each ultrasound pulse group. The processing for one frame FRB corresponds to processing of generating the phase inversion B-mode data corresponding to one phase inversion B-mode image.

In the transmission of the ultrasound pulse in the normal operation shown in Fig. 5, while the Doppler measurement information for one frame FR is being generated, the operation of alternately transmitting the ultrasound pulse groups is performed only once for the two adjacent transmission band pairs PTX1 and PTX2.

On the other hand, in the model application operation shown in Fig. 6, while the Doppler measurement information for one frame FR is being generated, the operation of alternately transmitting the ultrasound pulse groups is performed a plurality of times for the two adjacent transmission band pairs PTX1 and PTX2. In the example shown in Fig. 6, while the Doppler measurement information for one frame FR is being generated, the operation of alternately transmitting the ultrasound pulse groups is performed four times for the two adjacent transmission band pairs PTX1 and PTX2.

Therefore, the time interval at which the ultrasound pulse groups are transmitted in one frame FR is larger than that in Fig. 5, and the phase change of the plurality of reception Doppler signals obtained in one frame FR is sufficiently large. That is, the phase angle (the argument of a complex number) of the autocorrelation value A(r) shown in (Equation 2) is sufficiently large. Consequently, the measurement accuracy of the Doppler measurement information is increased. Further, for the two adj acent transmission band pairs PTX1 and PTX2, the ultrasound pulse groups are alternately transmitted, so that a region in which the Doppler measurement information is acquired per certain time is widened. Furthermore, the time required to generate the Doppler measurement information for one frame FR is shorter than that in Fig. 5, and the frame rate is increased.

Here, an embodiment has been described in which the ultrasound probe 14 comprises the plurality of transducer elements 16 arranged in a linear shape and the plurality of transmission bands are formed in a direction perpendicular to the arrangement direction of the transducer elements 16. The plurality of transducer elements 16 may be arranged in a curved shape at the tip part of the ultrasound probe 14. In this case, the plurality of transmission bands may be formed to extend radially from the tip part of the ultrasound probe 14.

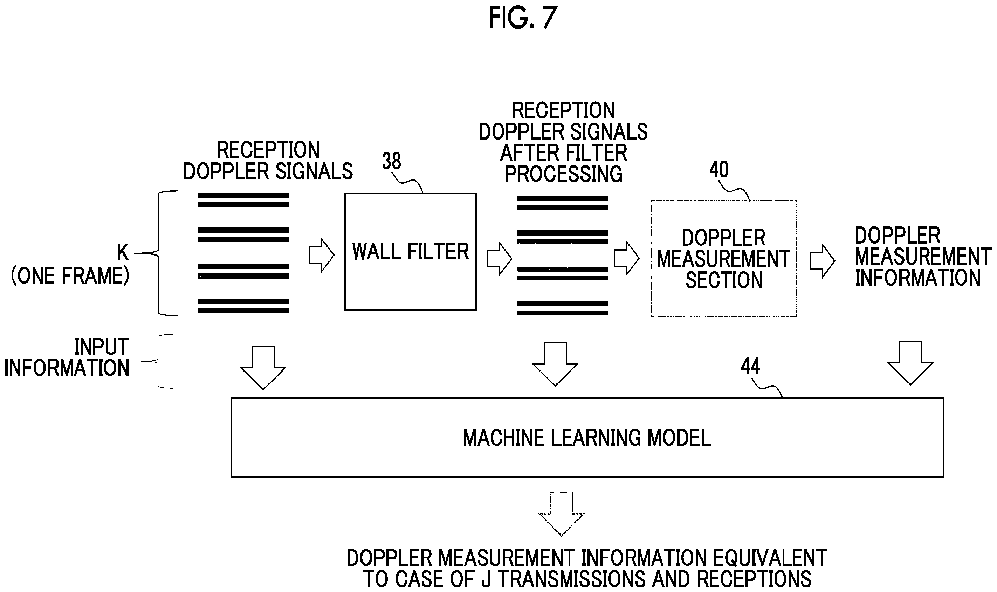

Fig. 7 shows the model application operation in a case in which the ultrasound pulses are transmitted from the ultrasound probe 14 in accordance with Fig. 6. Fig. 7 shows that the K reception Doppler signals before the high-pass filter processing is performed, the K reception Doppler signals after the high-pass filter processing is performed, and the Doppler measurement information are input to the machine learning model 44 once for the K transmissions and receptions of the series of ultrasound pulses for one frame FR. That is, as the K ultrasound pulses are transmitted from the ultrasound probe 14, and the K reflected waves are received by the ultrasound probe 14, the K reception Doppler signals are input to the wall filter 38 and the machine learning model 44. The wall filter 38 outputs the K reception Doppler signals after the high-pass filter processing to the Doppler measurement section 40 and the machine learning model 44. The Doppler measurement section 40 outputs the Doppler measurement information to the machine learning model 44 in response to the input of the K reception Doppler signals after the high-pass filter processing. In this way, the K reception Doppler signals, the K reception Doppler signals before the high-pass filter processing, the K reception Doppler signals after the high-pass filter processing, and the Doppler measurement information generated according to the K reception Doppler signals are input to the machine learning model 44 as the input information. The machine learning model 44 generates the Doppler measurement information equivalent to the case of the J transmissions and receptions based on the input information. For at least one of the time intervals between the adjacent reception pulse signals in the K reception Doppler signals, a time interval at which the Doppler frequency can be detected may be used.

The ultrasound pulse group PIB shown in Fig. 5 and the ultrasound pulse groups PIB1 to PIB8 shown in Fig. 6 may be utilized as ultrasound pulse groups not only for the B-mode but also for the Doppler mode. In this case, the reception pulse signal output from the phase alignment addition section 28 is output not only to the B-mode image generation section 32 but also to the quadrature detection section 36 in response to the transmission and reception of the ultrasound pulses belonging to the ultrasound pulse group PIB or the ultrasound pulse groups PIB1 to PIB8. For example, by using ultrasound pulses having the same waveform for the B-mode and the Doppler mode, the ultrasound pulses in the B-mode can be used in the Doppler mode. For the ultrasound pulses using the phase inversion, the quadrature detection section 36 gives a phase difference of 180° to two reception Doppler signals based on two ultrasound pulses.

The reception pulse signal as the phase-aligned reception signal (B-mode image reception signal) output from the reception unit 20 in response to the reflected wave for B-mode image generation is utilized for the operation in the Doppler mode as any of the K reception pulse signals output from the reception unit 20. The ultrasound pulse group PIB or the ultrasound pulse groups PIB1 to PIB8 are utilized as the ultrasound pulse groups not only for the B-mode but also for the Doppler mode, the effect equivalent to a case in which the number of transmissions and receptions of the ultrasound pulses is increased can be obtained in the operation of the Doppler mode.

Configuration of the Present Disclosure

Configuration 1: An ultrasound diagnostic apparatus comprising:

a transmission unit that transmits ultrasound pulses N times through an ultrasound probe, where N is an integer of 2 or more;
a reception unit that receives reflected waves generated N times in a measurement target object through the ultrasound probe; and
an information processing unit that generates N reception Doppler signals from N reception pulse signals output from the reception unit in response to the reflected waves generated N times in the measurement target object and that executes processing on each of the reception Doppler signals,
in which the information processing unit includes

a filter that performs high-pass filter processing on the N reception Doppler signals,
a Doppler measurement section that generates Doppler measurement information of the measurement target object based on the N reception Doppler signals that have been subjected to the high-pass filter processing, and
a machine learning model that is constructed based on training data,

under a condition that J and K are integers of 2 or more, with J being greater than K,
the training data includes

training information that is at least one of reception characteristic information, which is derived from each of the reception Doppler signals in a case in which N is set to K, or the Doppler measurement information, which is generated by the Doppler measurement section in a case in which N is set to K, and
target information that is at least one of the reception characteristic information, which is derived from each of the reception Doppler signals in a case in which N is set to J, or the Doppler measurement information, which is generated by the Doppler measurement section in a case in which N is set to J, for the same measurement target object as that in a case in which N is set to K, and

the machine learning model generates
the Doppler measurement information based on input information that is at least one of the reception characteristic information, which is derived from each of the reception Doppler signals in a case in which N is set to K, or the Doppler measurement information, which is generated by the Doppler measurement section in a case in which N is set to K, for a measurement target object in a subject.

Configuration 2: The ultrasound diagnostic apparatus according to Configuration 1, in which the reception characteristic information includes at least one of:

the N reception Doppler signals before the high-pass filter processing; or
the N reception Doppler signals after the high-pass filter processing.

Configuration 3: The ultrasound diagnostic apparatus according to Configuration 1 or 2,
in which the Doppler measurement information includes at least one of:

autocorrelation values of the N reception Doppler signals after the high-pass filter processing;
a velocity of the measurement target object obtained from the N reception Doppler signals after the high-pass filter processing;
a Doppler frequency variation degree for the N reception Doppler signals after the high-pass filter processing; or
a value indicating a magnitude of the N reception Doppler signals after the high-pass filter processing.

Configuration 4: The ultrasound diagnostic apparatus according to any one of Configurations 1 to 3,

in which the transmission unit transmits an ultrasound wave for B-mode image generation through the ultrasound probe,
the reception unit receives a reflected wave for B-mode image generation generated in the measurement target object through the ultrasound probe,
the information processing unit further includes
a B-mode image generation section that generates B-mode image data based on a B-mode image reception signal output from the reception unit in response to the reflected wave for B-mode image generation, and
the B-mode image reception signal output from the reception unit is utilized as any of the N reception pulse signals output from the reception unit in response to the reflected wave for B-mode image generation.

Configuration 5: The ultrasound diagnostic apparatus according to any one of configurations 1 to 4,

in which the transmission unit forms a plurality of transmission bands at different positions or in different directions for the ultrasound pulses,
for two adjacent transmission bands, the ultrasound pulses are alternately transmitted such that the ultrasound pulses are transmitted in one transmission band while no ultrasound pulses are transmitted in the other transmission band,
for two adjacent transmission band pairs, each including two adjacent transmission bands, with a series of ultrasound pulses alternately transmitted in two transmission bands included in one transmission band pair as an ultrasound pulse group, the ultrasound pulse groups are alternately transmitted such that the ultrasound pulse group is transmitted in one transmission band pair while no ultrasound pulse group is transmitted in the other transmission band pair, and
each time K ultrasound pulses are transmitted in each transmission band, the machine learning model generates the Doppler measurement information for one frame for each transmission band.

Configuration 6: The ultrasound diagnostic apparatus according to Configuration 5,
in which, while the Doppler measurement information for one frame is being generated, an operation of alternately transmitting the ultrasound pulse groups is performed a plurality of times for the two adjacent transmission band pairs.

Explanation of References

10: beam control unit
12: transmission unit
14: ultrasound probe
16: transducer element
18: subject
20: reception unit
22: control unit
24: operation unit
26: information processing unit
28: phase alignment addition section
32: B-mode image generation section
34: image processing section
36: quadrature detection section
38: wall filter
40: Doppler measurement section
42: machine learning section
44: machine learning model
46: display unit
100: ultrasound diagnostic apparatus

**Claims**

1. An ultrasound diagnostic apparatus (100) comprising:

a transmission unit (12) that transmits ultrasound pulses N times through an ultrasound probe (14), where N is an integer of 2 or more;

a reception unit (20) that receives reflected waves generated N times in a measurement target object through the ultrasound probe (14); and

an information processing unit (26) that generates N reception Doppler signals from N reception pulse signals output from the reception unit (20) in response to the reflected waves generated N times in the measurement target object and that executes processing on each of the reception Doppler signals,

wherein the information processing unit (26) includes

a filter (38) that performs high-pass filter processing on the N reception Doppler signals,

a Doppler measurement section (40) that generates Doppler measurement information of the measurement target object based on the N reception Doppler signals that have been subjected to the high-pass filter processing, and

a machine learning model (44) that is constructed based on training data,

under a condition that J and K are integers of 2 or more, with J being greater than K,

the training data includes

training information that is at least one of reception characteristic information, which is derived from each of the reception Doppler signals in a case in which N is set to K, or the Doppler measurement information, which is generated by the Doppler measurement section (40) in a case in which N is set to K, and

target information that is at least one of the reception characteristic information, which is derived from each of the reception Doppler signals in a case in which N is set to J, or the Doppler measurement information, which is generated by the Doppler measurement section (40) in a case in which N is set to J, for the same measurement target object as that in a case in which N is set to K, and

the machine learning model (44) generates

the Doppler measurement information based on input information that is at least one of the reception characteristic information, which is derived from each of the reception Doppler signals in a case in which N is set to K, or the Doppler measurement information, which is generated by the Doppler measurement section (40) in a case in which N is set to K, for a measurement target object in a subject.

2. The ultrasound diagnostic apparatus (100) according to claim 1,
   wherein the reception characteristic information includes at least one of:

   the N reception Doppler signals before the high-pass filter processing; or
   the N reception Doppler signals after the high-pass filter processing.

3. The ultrasound diagnostic apparatus (100) according to claim 1 or 2,
   wherein the Doppler measurement information includes at least one of:

   autocorrelation values of the N reception Doppler signals after the high-pass filter processing;
   a velocity of the measurement target object obtained from the N reception Doppler signals after the high-pass filter processing;
   a Doppler frequency variation degree for the N reception Doppler signals after the high-pass filter processing; or
   a value indicating a magnitude of the N reception Doppler signals after the high-pass filter processing.

4. The ultrasound diagnostic apparatus (100) according to any one of claims 1 to 3,

   wherein the transmission unit (12) transmits an ultrasound wave for B-mode image generation through the ultrasound probe (14),
   the reception unit (20) receives a reflected wave for B-mode image generation generated in the measurement target object through the ultrasound probe,
   the information processing unit (26) further includes
   a B-mode image generation section (32) that generates B-mode image data based on a B-mode image reception signal output from the reception unit (20) in response to the reflected wave for B-mode image generation, and
   the B-mode image reception signal output from the reception unit (20) is utilized as any of the N reception pulse signals output from the reception unit (20) in response to the reflected wave for B-mode image generation.

5. The ultrasound diagnostic apparatus (100) according to any one of claims 1 to 4,

wherein the transmission unit (12) forms a plurality of transmission bands at different positions or in different directions for the ultrasound pulses,

for two adjacent transmission bands, the ultrasound pulses are alternately transmitted such that the ultrasound pulses are transmitted in one transmission band while no ultrasound pulses are transmitted in the other transmission band,

for two adjacent transmission band pairs, each including two adjacent transmission bands, with a series of ultrasound pulses alternately transmitted in two transmission bands included in one transmission band pair as an ultrasound pulse group, the ultrasound pulse groups are alternately transmitted such that the ultrasound pulse group is transmitted in one transmission band pair while no ultrasound pulse group is transmitted in the other transmission band pair, and

each time K ultrasound pulses are transmitted in each transmission band, the machine learning model (44) generates the Doppler measurement information for one frame for each transmission band.

6. The ultrasound diagnostic apparatus (100) according to claim 5,

wherein, while the Doppler measurement information for one frame is being generated, an operation of alternately transmitting the ultrasound pulse groups is performed a plurality of times for the two adjacent transmission band pairs.

FIG. 1

100

- 14 ULTRASOUND PROBE
- 16
- 18 SUBJECT
- 12 TRANSMISSION UNIT
- 10 BEAM CONTROL UNIT
- 20 RECEPTION UNIT
- 24 OPERATION UNIT
- 22 CONTROL UNIT
- 26
- 28 PHASE ALIGNMENT ADDITION SECTION
- 32 B-MODE IMAGE GENERATION SECTION
- 34 IMAGE PROCESSING SECTION
- 46 DISPLAY UNIT
- 36 QUADRATURE DETECTION SECTION
- 38 WALL FILTER
- 42 MACHINE LEARNING SECTION
- 44 MACHINE LEARNING MODEL
- 40 DOPPLER MEASUREMENT SECTION

# FIG. 2

# FIG. 3

RECEPTION
DOPPLER SIGNALS

38

RECEPTION
DOPPLER SIGNALS
AFTER FILTER
PROCESSING

40

J

WALL FILTER

DOPPLER
MEASUREMENT
SECTION

DOPPLER
MEASUREMENT
INFORMATION

TARGET
INFORMATION

RECEPTION
CHARACTERISTIC
INFORMATION

42

MACHINE LEARNING SECTION

EP 4 575 569 A2

# FIG. 4

RECEPTION
DOPPLER SIGNALS

RECEPTION
DOPPLER SIGNALS
AFTER FILTER
PROCESSING

38

40

K { ▤▤▤ ⇨  | WALL FILTER | ⇨ ▤▤ ⇨ | DOPPLER MEASUREMENT SECTION | ⇨  DOPPLER MEASUREMENT INFORMATION

INPUT
INFORMATION {

⇩  ← RECEPTION CHARACTERISTIC → ⇩
INFORMATION

44

⇩

| MACHINE LEARNING MODEL |

⇩

DOPPLER MEASUREMENT INFORMATION EQUIVALENT
TO CASE OF J TRANSMISSIONS AND RECEPTIONS

EP 4 575 569 A2

FIG. 5

FIG. 6

# FIG. 7

RECEPTION DOPPLER SIGNALS

K (ONE FRAME)

38

WALL FILTER

RECEPTION DOPPLER SIGNALS AFTER FILTER PROCESSING

40

DOPPLER MEASUREMENT SECTION

DOPPLER MEASUREMENT INFORMATION

INPUT INFORMATION

44

MACHINE LEARNING MODEL

DOPPLER MEASUREMENT INFORMATION EQUIVALENT TO CASE OF J TRANSMISSIONS AND RECEPTIONS

EP 4 575 569 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005102718 A
- JP 8154935 A
- JP H08154935 A
- JP 2016087302 A

- JP 2009005737 A
- JP 7016227 A
- JP H0716227 A
- JP 2004532711 A